# EUROPEAN PATENT APPLICATION

(11) **EP 4 628 092 A1**
(43) Date of publication of application: **08.10.2025**
(21) Application number: 23901199.2
(22) Date of filing: 30.11.2023
(51) Int. Cl.: A61K 38/28, A61K 31/455, A61K 31/195, A61P 3/10

(54) **INSULIN ASPART COMPOSITION (EMBODIMENTS)**

(30) Priority: 04.12.2022 RU 2022131585
(71) Applicant: OOO "Geropharm", Sankt-Peterburg, 191119 (RU)
(72) Inventor: SHITIKOVA, Viktoriya Olegovna, Sankt-Peterburg, 190005 (RU); YUDAEVA, Nina Valeryevna, Sosnovyy Bor, 188540 (RU)
(74) Representative: Koitel, Raivo
(86) International application number: PCT/RU2023/050280
(87) International publication number: WO 2024/123214

(57) **Abstract**

The present invention relates to the field of pharmacy and medicine, *i.e.,* to compositions of fast-acting injectable insulin preparation. More specifically, the present invention relates to the compositions comprising insulin aspart, a nicotinic acid compound, a surfactant selected from poloxamer 188, polysorbate 20, polysorbate 80, or a combination thereof, an amino acid selected from lysine, arginine and/or a salt thereof, and/or a combination thereof. The compositions of the invention are useful for reducing blood glucose level in a subject, and are particularly useful in insulin preparations requiring high stability against thermal and/or physical and mechanical stress.

## Description

### Field of the invention

The present invention relates to the field of pharmacy and medicine, *i.e.,* to the compositions of fast-acting injectable insulin preparation for reducing blood glucose level in a subject. More specifically, the present invention relates to compositions comprising insulin aspart, wherein the compositions have excellent chemical and physical stability, in particular, upon exposure to high temperatures and/or high mechanical energy.

### Background of the invention

Pharmaceutical preparation (PP) stability is essential to ensure a therapeutic effect and the absence of new adverse reactions, and should be maintained during both PP shelf life and its use. Stable PP compositions are especially crucial when used in delivery devices where they are exposed to elevated temperatures and/or mechanical stress. For example, stable insulin compositions are required for using in injection pens and continuous (pump-assisted) infusion systems. Further, insulin compositions are manufactured, stored and used in vials that requires their stability throughout the shelf life.

Injection pens are widely used by patients suffering from diabetes, the so-called diabetic persons, since such pens allow them to manage the disease independently and efficiently by just-in-time injecting a drug at required doses into a target site on the patient's body, exactly in accordance with physician's prescriptions. Such injection pens are quite easy to operate, so the users do not need any special medical skills and/or any special medical education in order to set a required dose of insulin for injection, and to subsequently inject the set drug dose to a target injection site on their body. Injection pens usually comprise a cartridge containing a certain amount of PP. The cartridge includes a plunger and a mechanism for advancing the plunger inside the cartridge so as to allow to dose the drug. Injection pens may be reusable or disposable. In reusable pens, a user can replace the spent cartridge and put the pen's actuator screw back to initial position. In a disposable injection pen, the cartridge is not replaceable; the pen is discarded after the cartridge contents are spent. Insulin preparation, when used in cartridges or carried within replacement cartridges, is stored outside refrigerator until either the cartridge is used up (usually within a period of at least 2 weeks), or until the end of shelf life period recommended by the manufacturer for the cartridges being in use or for replaceable cartridges, i.e., within 1 month. Since insulin injection pens are often stored in clothing pockets at temperatures close to body temperature, insulin compositions used in such pens are exposed to physical and thermal stress, and therefore have limited stability.

For continuous infusion systems, insulin solution to be administered by the infusion device is stored in a reservoir, e.g., a syringe, a synthetic polymer chamber, a metal container, or the like. The reservoir and a pumping mechanism operably connected thereto are attached to or implanted into patient's body. Insulin is pumped from a reservoir through small-diameter catheters subcutaneously, intravenously, or intraperitoneally; the insulin composition is thus exposed to body temperature and movement, as well as forced pump-assisted motion, and, hence, to high thermomechanical stress.

Existing insulin compositions, mostly comprising monomeric insulin analogues, fail to provide sufficient physicochemical stability in injection pens and continuous infusion systems. The main problem related to insulin administration via injection pens and infusion systems is the tendency of insulin solutions to form aggregates, fibrils, or insulin precipitates over time [1]. Aggregates and precipitates damage components of catheters and pumps, and, in case of injection pens, damage needles, which contributes to cessation of insulin delivery to patient's body and to poor glycemic control.

Many factors influence aggregation and precipitation of insulin in solution, the main ones being the following:
(a) elevated temperature, for example, 25-37°C, as opposed to normal (2-8°C) storage conditions [2];
(b) mechanical energy caused by motion of a body, or by motion of pump/actuator mechanisms [3];
(c) prolonged interaction of insulin molecules with hydrophobic surfaces such as air interfaces, and plastic or metal pump components [4,5].

Insulin is a peptide hormone regulating blood glucose level in mammals by initiating a signaling cascade that accelerates glucose uptake and glycogen production upon binding to the insulin receptor. When insulin production is compromised, glucose concentration in blood increases (chronic hyperglycemia), which is the primary diagnostic marker for type 1 diabetes (T1DM). However, in cases where signaling through insulin receptors is impaired, type 2 diabetes (T2DM) develops even when there is enough insulin hormone produced, which is a result of decreased tissue susceptibility to the action of insulin (insulin resistance). The efficiency of insulin in the treatment of diabetes due to the ability of this hormone to reduce blood glucose levels has been proven over decades of medical use. However, the demand for novel approaches to the treatment of diabetes mellitus is still actual due to the increased incidence of the disease and the need of considering patients' individual features and preferences [6].

Development of fast-acting and superfast-acting insulin analogues, having a faster onset of action and shorter duration of action, has become a significant advance in the treatment of type 1 and type 2 diabetes. In a neutral solution, at pharmaceutical concentrations, insulin and insulin analogues occur in the form of stabilized zinc-containing hexamers consisting of three identical dimeric units. The delay in insulin action is largely due to the time it takes for the hexamers to dissociate into monomers and dimers which are capable of being absorbed [7,8]. The first class of fast-acting insulins (e.g., insulin lispro and aspart) was developed based on amino acid mutations in the human insulin sequence resulting in a weakened self-association of insulin oligomers. For example, replacing proline amino acid at position B28 with aspartic acid in insulin aspart reduces the molecules' tendency to form hexamers which is observed in a solution of soluble human insulin. Correspondingly, insulin aspart is absorbed from subcutaneous adipose tissue much faster compared to soluble human insulin, and is widely used for postprandial blood glucose control in diabetes mellitus patients [9].

Fast-acting insulin analogues in monomeric or dimeric form are known to have reduced physicochemical stability compared to hexamers [10], especially under thermal and mechanical stress, in particular, they form high molecular weight impurities and strongly tend to form aggregates (fibrillation), which manifests as turbidity and precipitate. High molecular weight impurities (dimers, trimers, and polymers) and aggregates cut down the administered dose of insulin, and, moreover, can encourage irritation or immune reactions in a patient. Also, insoluble aggregates can clog and damage needles, cannulas, and pump tubing. To ensure the quality of insulin composition, it is necessary to prevent formation of polymers and aggregates.

Insulin aspart preparations are commercially available (for example, NovoRapid^{®} (Novo Nordisk A/S), RinFast^{®} (GEROPHARM OOO). It has been shown that overall glycemic control can be improved by supplementing the preparations with auxiliary substance - nicotinamide - able to accelerate absorption of insulin aspart compared to insulin aspart preparations lacking nicotinamide. Specifically, Fiasp (comprising nicotinamide) was shown to provide improved overall glycemic control and better postprandial glycemic control without increasing overall risk of severe or confirmed hypoglycemia in T1DM and T2DM patients as compared to NovoRapid (lacking nicotinamide). Pharmacokinetics of the fast-acting insulin aspart Fiasp may better mimic rapid endogenous secretion of prandial insulin and thus provide improved postprandial glycemic control compared with insulin aspart [11].

Nicotinamide can accelerate insulin absorption (WO 91/09617), including insulin aspart absorption (WO/9610417), however, it has a negative effect on chemical stability by increasing the content of impurities (RU2533217). The chemical stability of the Fiasp pharmaceutical preparation (PP) is enhanced by introduction of arginine, which can be seen as a decrease in dimeric and polymeric content and deamidated insulin content during storage (RU2533217). FIASP is the closest prior art of the present invention.

A report from a small scale (n = 37) 6-week study of the infusion system susceptibility to clogging and malfunction reveals no observed cases of infusion system occlusions with either FIASP (25 subjects) or insulin aspart (12 subjects). However, unexplained hyperglycemia and early replacement of infusion sets were more common with FIASP compared to insulin aspart [12].

Patent RU2533217 discloses *(see* example 2) that arginine, when included into the compositions containing insulin aspart and nicotinamide, reduces amounts of forming degradation products, especially high molecular weight proteins and deamidated forms, however, at the same time it reduces physical stability measured as lag time in the thioflavin T assay, and the physical stability continues to decline as the arginine concentration increases.

Our study of the fibrillation tendency in the thioflavin T assay also showed that FIASP is more prone to fibrillation compared to the NovoRapid.

Thus, there remains a current need for insulin compositions having enhanced stability when used in injection pens or during continuous pump-assisted infusion, i.e., from several days to several months. Enhanced stability of insulin compositions when used in vials will also be beneficial. The above-described technical issue is especially relevant for compositions of monomer insulin analogues, including those of insulin aspart, which have lower stability compared to hexameric forms of insulin, and for compositions comprising nicotinic acid compounds that can accelerate absorption of insulin, but affect its chemical stability.

### SUMMARY OF THE INVENTION

We unexpectedly found that including surfactants such as poloxamer 188, polysorbate 20, polysorbate 80, or combinations thereof, combined with one or more amino acids such as lysine, arginine and/or salts thereof and/or combinations thereof, into compositions comprising insulin aspart and nicotinamide improves both chemical and physical stability of the insulin aspart compared to the closest prior art.

The present invention relates to compositions for reducing blood glucose level in a subject, the compositions comprising insulin aspart (B28Asp), a nicotinic acid compound, one or more amino acids or a salt thereof selected from lysine, arginine or salts thereof, and a surfactant.

In preferred embodiments, the present invention relates to compositions for reducing blood glucose levels in a subject, the compositions comprising insulin aspart, a nicotinic acid compound, an amino acid selected from lysine, arginine and/or a salt thereof and/or a combination thereof, and a surfactant selected of poloxamer 188, polysorbate 20, polysorbate 80 and/or combinations thereof.

The concentration of insulin aspart in the compositions of the invention ranges from about 0.2 mM and about 2.0 mM (from about 33 U/ml to about 333 U/ml), preferably from about 0.3 mM to about 1.2 mM (from about 50 U/ml to about 200 U/ml), most preferably is 0.6 mM (100 U/ml).

In one embodiment of the present invention, the compositions have a pH of 6.5 to 8.5, preferably 6.6 to 7.4, even more preferably pH is 7.

The compositions of the invention contain a nicotinic acid compound selected from nicotinamide (niacinamide), nicotinic acid (niacin) and/or a salt thereof and/or any combination thereof. The concentration of nicotinamide or other nicotinic acid compound in the compositions of the invention is from about 1 mM to about 200 mM. Preferable nicotinic acid compound is nicotinamide.

Concentrations of lysine and/or a salt thereof, and arginine and/or a salt thereof, or total concentrations of lysine and/or a salt thereof in combination with arginine and/or a salt thereof range from about 1 mM to about 100 mM.

In compositions comprising a combination of lysine and/or a salt thereof and arginine and/or a salt thereof, the molar ratio of the amino acids is from 4000:1 to 1:4000, respectively, said range including each and every integer ratio, *e.g.,* 100:1, 99:1, 98:1, *etc.* In one preferred embodiment, the lysine:arginine molar ratio is 1:1, 4:5, 5:4, 6:4; 5:2, or 2:5.

The compositions of the invention may further comprise protease inhibitor(s), metal ions, buffer systems, pH adjusting agents, preserving agent(s), isotonic agent(s), chelating agent(s), stabilizers and surfactants.

In one embodiment, the compositions of the invention are aqueous compositions, i.e., they comprise water and occur as solutions or suspensions.

The compositions of the present invention can be used for the treatment or prevention of hyperglycemia, type 2 diabetes mellitus, impaired glucose tolerance, and type 1 diabetes mellitus.

In one preferred embodiment, the compositions of the invention are administered parenterally. Parenteral administration can be performed by subcutaneous, intramuscular, intraperitoneal, or intravenous injection using a syringe, which is optionally an injection pen. Alternatively, parenteral administration can be performed using an infusion pump.

Accordingly, the present invention further relates to injectable insulin preparations comprising compositions of the present invention.

The compositions of the invention showed better chemical and physical stability compared to the closest prior art, which is beneficial for consumers, especially in cases where injection pens or insulin pumps are used.

### TERMS AND DEFINITIONS

As used herein, the term "human insulin" means a human hormone having well-known structure and properties. Insulin is a 51 amino acid polypeptide divided up into 2 amino acid chains: A-chain, consisting of 21 amino acids, and B-chain, consisting of 30 amino acids. The chains are interconnected with 2 disulfide bridges. For many years, insulin pharmaceutical preparations have been used to treat diabetes mellitus, and recently not only natural insulins are used but also insulin derivatives and analogues.

As used herein, the term "insulin analogue" means a polypeptide derived from the primary structure of naturally occurring insulin, such as human insulin, by mutation. One or more mutations are obtained by deletion and/or substitution of at least one amino acid residue occurring in the natural insulin and/or by addition of at least one amino acid residue thereto. Mutations in the insulin molecule are indicated by the respective chain reference (A or B), a respective position number, and a three-letter code for the amino acid replacing the native one.

The term "fast-acting insulin" or "short-acting insulin" refers to the insulin analogues and/or insulin derivatives have an onset of action within 5-15 minutes and duration of action that lasts for 3-4 hours. Examples of fast-acting insulins include, but are not limited to the following: insulin aspart, insulin lispro and insulin glulisine.

The terms "monomeric human insulin analogue" and "monomeric insulin analogue" are well known in the art and usually refer to the fast-acting human insulin analogues. They include, for example, insulin lispro (Humalog^{®}), insulin aspart (NovoRapid^{®}), and insulin glulisine (Apidra^{®}). The molecular structure of insulin lispro is identical to that of human insulin except for positions 28 and 29 in the B-chain of the molecule, where lysine and proline are arranged in reverse order (human insulin: B28ProB29Lys, lispro: B28LysB29Pro). The reverse lysine/ proline arrangement allows the lispro molecule to dissociate 2 times faster. In insulin aspart (B28Asp), proline amino acid residue at position 28 of the B-chain is replaced with Asp, reducing the molecules' tendency to form hexamers which is observed in human insulin solution. In the insulin glulisine structure (3BLys29BGlu) asparagine amino acid residue at position 3 of the B-chain in human insulin molecule is replaced by lysine, and lysine 29 of the B-chain is replaced by glutamine, which contributes to stability of the preparation in solution as monomeric and dimeric forms.

The content of human B28Asp insulin in solution may range from about 0.2 mM to about 2.0 mM (between about 33 and about 333 international units (IU)/ml), preferably from about 0.3 mM to about 1.2 mM (from about 50 to about 200 IU/ml), most preferably the concentration is 0.6 mM (100 U/ml), in injectable preparations. However, the insulin compound content may be higher for parenteral administration performed with other purposes.

In this context, the IU unit corresponds to 6 nmol.

Herein, the term "insulin aspart composition" means a product comprising insulin aspart, a nicotinic acid compound selected from nicotinamide (niacinamide), nicotinic acid (niacin) and/or a salt thereof and/or any combination thereof, an amino acid selected from lysine, arginine and/or salts thereof and/or combinations thereof, a surfactant selected from poloxamer 188, polysorbate 20, polysorbate 80 or a combination thereof, which product may further comprise other excipients such as preservatives, chelating agents, isotonic agents, fillers, stabilizers, antioxidants, polymers and surfactants other than the above-mentioned ones, metal ions, oil carriers and proteins (e.g., human serum albumin, gelatin or proteins), pH adjusting agents, wherein the insulin aspart composition can be used for the treatment, prevention or reduction the severity of a disease or disorder by administering the composition to a human. The composition of the present invention may thus be referred to as a "pharmaceutical composition" or a "pharmaceutical preparation".

The terms "protein preparation", "protein composition", and "protein" can be used interchangeably herein meaning any drug comprising, as an active substance, a molecule comprised of the polypeptide chains consisting of amino acid residues linked linearly by peptide bonds, independently or in combination with other compounds. Insulin composition or insulin, for example, are particular cases and are encompassed by these terms.

The terms "lysine" and "arginine" refer to amino acids and include **D-** and L-enantiomers, as well as mixtures thereof. The term also includes any pharmacologically acceptable salts of lysine and arginine. Lysine and arginine readily form salts, such as hydrochlorides.

As used herein, the term "surfactant" (surface-active compound, SAC) refers to any molecules or ions that consist of a water-soluble (hydrophilic) portion, a head portion, and a fat-soluble (lipophilic) segment. Surfactants accumulate preferably at the interface, with the hydrophilic part oriented towards water (hydrophilic phase), and the lipophilic part oriented towards oil or hydrophobic phase (i.e., glass, air, oil, etc.). Surfactants reduce the surface tension of liquids. They are also known as amphipathic compounds. The term "detergent" is generally synonymous with the term "surfactant". The use of surfactants in pharmaceutical preparations is well known to those skilled in the art. Such surfactants include, for example, polysorbate 20, polysorbate 80, poloxamer 188, and the like; these substances are described in the current Pharmacopoeias (USP, EP) as excipients.

The term "nicotinic acid compound" includes nicotinamide (niacinamide), nicotinic acid (niacin) and/or salts thereof and/or any combination thereof.

The buffer may be selected from the group including but not limited to sodium acetate, sodium carbonate, citrate, sodium dihydrogen phosphate, sodium hydrogen phosphate, sodium phosphate; and tris(hydroxymethyl)aminomethane, bicine, tricine, malic acid, succinate, maleic acid, fumaric acid, tartaric acid, aspartic acid, or mixtures thereof. Each of these specific buffers, and each of combinations thereof makes an alternative embodiment of the invention.

The compositions of the invention may further comprise other ingredients typically used in insulin preparations, e.g., zinc complexing agents.

Isotonic agents, such as glycerol, mannitol, sodium chloride, dextrose, and the like, may also be present in the compositions of the invention. An isotonic agent is a physiologically acceptable compound that imparts suitable tonicity to a composition, preventing diffusion of water across cell membranes contacting with a pharmaceutical composition.

The compositions of the invention may comprise a pharmaceutically acceptable preserving agent. The preserving agent present in the insulin preparation of the invention may be phenol, m-cresol, methylparaben, *etc.*

The compositions of the present invention may further comprise a chelating agent. The use of chelating agents in pharmaceutical preparations is well known to those skilled in the art.

The compositions of the invention may further comprise a stabilizer. As used herein, the term "stabilizer" refers to the chemicals added to pharmaceutical preparations comprising a polypeptide for stabilizing said polypeptide, i.e. for extending the shelf life and/or usage period of such preparations.

As used herein, the term "stability" refers to the chemical and physical stability of compositions with monomeric insulin analogues.

Physical instability of a protein composition (protein preparation) and/or a protein may result from aggregation of the protein molecules leading to formation of higher order polymers or even precipitates. Physical denaturation of insulin is known as fibrillation. **In** the fibrillar state, elongated peptide chains are arranged in parallel or antiparallel orientation and are interconnected via hydrogen bonds, forming the so-called β-structure or β-sheets. Fibrils typically represent the lowest energy state of a protein; regeneration of the protein from this state to the native properly folded state is only possible in a strong alkaline environment. Insulin fibrils take the form of gels or precipitates. Exposure to thermomechanical stress and/or interaction with interphase boundaries and hydrophobic surfaces are the factors promoting acceleration of fibril formation. Fibrillation is believed to occur due to insulin monomerization. Monomeric insulin analogues, which readily dissociate from hexameric units to the monomeric form, are more likely to form fibrils compared to human insulin.

Physical stability can be assessed by methods well known in the art, for example, by measuring turbidity (optical density). Turbidity results from aggregation or precipitation of proteins or complexes in compositions.

The physical stability of aqueous protein preparations can also be assessed by using a spectroscopic agent or a probe of the conformational status of the protein. The probe is preferably a small molecule that predominantly binds to non-native protein conformers. One example of a small molecule probe for spectroscopic protein structure determination is thioflavin T. Thioflavin T is a fluorescent dye widely used for detection of amyloid fibrils. **In** the presence of fibrils and possibly some other protein configurations, thioflavin T produces a new excitation maximum at approximately 445 nm and enhances emission at approximately 485 nm upon binding to the fibrillar form of the protein. Unbound thioflavin T is substantively non-fluorescent at these wavelengths.

As used herein, the term "chemical stability" in relation to a protein composition (protein preparation) and/or a protein refers to changes in the protein covalent structure resulting in formation of chemical degradation products with potentially lower biological activity and/or potentially enhanced immunogenic properties compared to native protein structure. Various chemical degradation products may form depending on the type and nature of native protein, and on environmental factors the protein is exposed to. An increase in chemical degradation products is commonly observed during storage and use of a protein preparation. Most proteins are prone to deamidation; other degradation pathways involve formation of high molecular weight products, where two or more protein molecules covalently bind to each other via transamidation and/or disulfide bonds, resulting in the covalently linked dimeric, oligomeric, and polymeric degradation products [13]. Another chemical degradation variant worth mentioning is oxidation (e.g., oxidation of methionine residues). The chemical stability of a protein preparation and/or a protein can be assessed by measuring the amount of chemical degradation products at different time points after exposure to various environmental conditions (formation of degradation products can often be accelerated, for example, by increasing the temperature). The amount of each individual degradation product is commonly determined by separating the degradation products based on their molecular size and/or charge using various chromatography techniques (e.g., size exclusion liquid chromatography (SEC-HPLC), and/or reverse-phase high-performance liquid chromatography (RP-HPLC)). Since high molecular weight protein products are potentially immunogenic and biologically inactive, HMWP levels must be kept low.

A "stable composition" is a composition wherein the extent of protein aggregation and impurities content fall within normal limits and do not exceed these limits over time.

In one embodiment, the compositions of the invention are aqueous compositions, i.e., compositions that comprise water and occur as solutions or suspensions.

The term "aqueous composition" refers to a composition comprising water. The terms "aqueous solution" and "aqueous suspension" refer to a solution or suspension comprising water, respectively. Aqueous suspensions may comprise active compounds in admixture with excipients suitable for preparing the aqueous suspensions.

### EXAMPLES

### Example 1. Preparing compositions 1-9.

In one embodiment, the pharmaceutical compositions of the invention are prepared as aqueous solutions. Table 1 shows the qualitative and quantitative content of some of the prepared compositions. The compositions of the invention include, but are not limited to compositions 2, 3, 6-9 listed in Table 1.

The method for producing the compositions comprises:
a) preparing an insulin aspart solution by dissolving the insulin aspart in water or a buffer;
b) preparing a zinc salt solution by dissolving the zinc salt in water or a buffer;
c) preparing a solution of preserving agents (phenolic compounds) by dissolving them in water or a buffer;
d) preparing an isotonic agent solution by dissolving the isotonic agent in water or a buffer;
e) preparing a nicotinamide solution and/or a solution of other nicotinic acid compound by dissolving the same in water or a buffer;
f) preparing a surfactant solution by dissolving the surfactant in water or a buffer;
g) preparing a solution of lysine and/or arginine by dissolving the amino acids and/or salts thereof in water or a buffer;
h) mixing Solution a) and Solutions b), c), d), e), f), g) in accordance with the composition formulations given in Table 1;
i) adjusting pH value of the mixture h) to 6,6-7,4, followed by sterilizing filtration.
j) aseptic filling the prepared compositions into cartridges.

Compositions of the invention can be obtained using any other method.

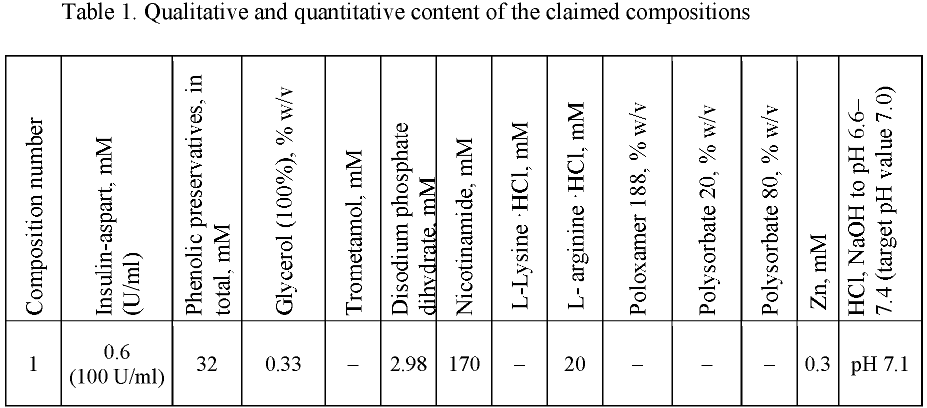

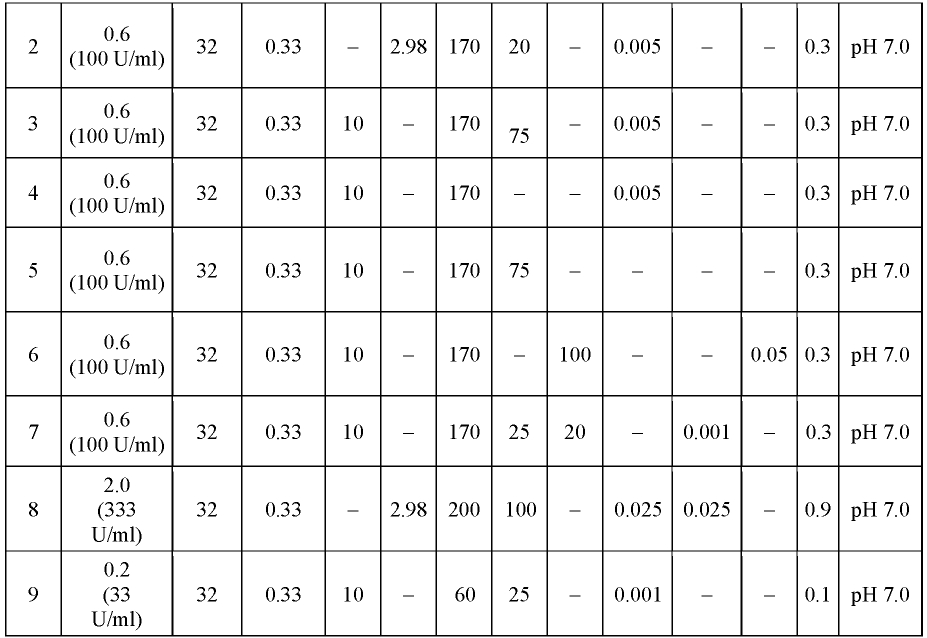

### Example 2. Analysis of physical and chemical stability of the compositions

Chemical stability was determined for compositions 1-9, prepared as in Example 1, after thermostatting in aseptically filled cartridges at 40° C for 1 and 2 weeks.

Insulin aspart-related impurities were determined using HPLC with UV detection and "Quantification" option in a column packed with octadecyl silica gel having 5 µm particle size and 300 Å pore size, with a mobile phase flow rate of 1.0 ml/min, at 214 nm wavelength.

Elution was carried out in a gradient mode using mobile phase comprised of the following components:
*mobile phase A (MP A):* 10 % (w/V) acetonitrile, anhydrous sodium sulfate buffer solution pH 3,4 (1,4 % (w/V), 0,13 % (V/V) concentrated orthophosphoric acid, 2 M sodium hydroxide to pH 3,4 ± 0,05).
*mobile phase B (MP B):* 50 % (w/V) acetonitrile.

B28IsoAsp, deamidated forms (B3iso, A21Asp, B3Asp), and other related impurities were quantified using intemal normalization method, by the absorption area of the corresponding peaks measured as a % of the peak area sum for the peaks which eluted after nicotinamide and preservatives.

High molecular weight protein (HMWP) was quantified by size exclusion liquid chromatography in a column packed with 10 µm particle size, 125 Å pore diameter hydrophilic chromatography silica gel. A mixture of acetonitrile: glacial acetic acid: 0.1% arginine solution (20:15:65) (V/V/V) was used as an eluent. Elution was carried out with a flow rate of 0.5 ml/min at a detection wavelength of 276 nm.

The HMWP content was determined using internal normalization method, by the sum of absorption areas for all peaks having shorter retention times compared to the insulin aspart monomer peak. Peaks having longer retention times than that of the insulin aspart monomer *(i.e.,* nicotinamide and preservatives) were excluded from the calculation.

The physical stability of the protein compositions was evaluated by testing the tendency for fibrillation in the Thioflavin-T (ThT) assay. The chosen method is described in literature, e.g., in RU2533217. The tendency for forming fibrils was determined for compositions 1-9 of Example 1, and for original Fiasp as a finished pharmaceutical product. Composition 1 was similar to the Fiasp composition. The compositions were assayed immediately after preparation.

### Preparation of the solutions

### Thioflavin stock solution

A precise portion of the dry Thioflavin T reagent is dissolved in methanol to the concentration of 1 mg/ml (corresponding to 3.14 mM). The solution is thoroughly vortexed and stored protected from light at ± 2 - 8 °C for up to 6 months.

### Thioflavin working solution

A working 200 µM thioflavin solution is prepared in purified water. The volume ratio of the stock solution to purified water is adjusted to 1:14.7.

The freshly prepared solution is used immediately.

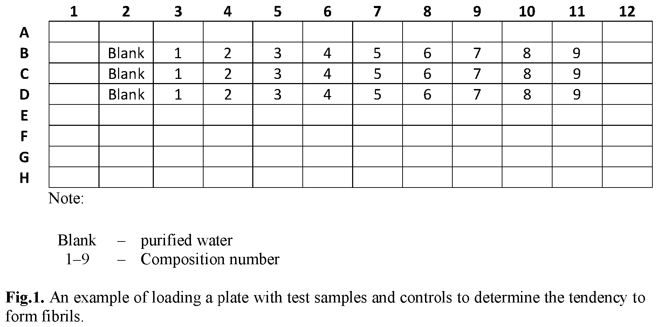

100 µl sample aliquots in triplicates are placed in a black 96-well plate, and purified water is used as a control sample. 10 µl of the thioflavin working solution was added to all wells. The edge wells are not used when filling the plate (see Fig. 1). The plate is sealed with transparent film and placed in CLARIOstar multi-mode plate reader (BMG, Germany). The fluorescent signal is detected every 20 minutes for 12 hours at a wavelength of 445/485 nm. Between readings, the plate is stirred at 700 rpm. Stirring and detection are performed at 37 °C.

Based on the measured results, the signal in relative fluorescence units is plotted against time in hours for each of the samples. A 4-parameter model is used for analysis.

The graph is used to determine T1/2max, *i.e.,* the time required for half of the analyzed sample to aggregate. When the graph takes the form of a straight line at zero value level, the sample is considered to be not prone to aggregation; in other cases, tendency to aggregation is measured by T1/2max.

Along with T1/2max, the ThT fluorescence curve is used to visually determine the lag time as the time point where ThT fluorescence differs from the background level.

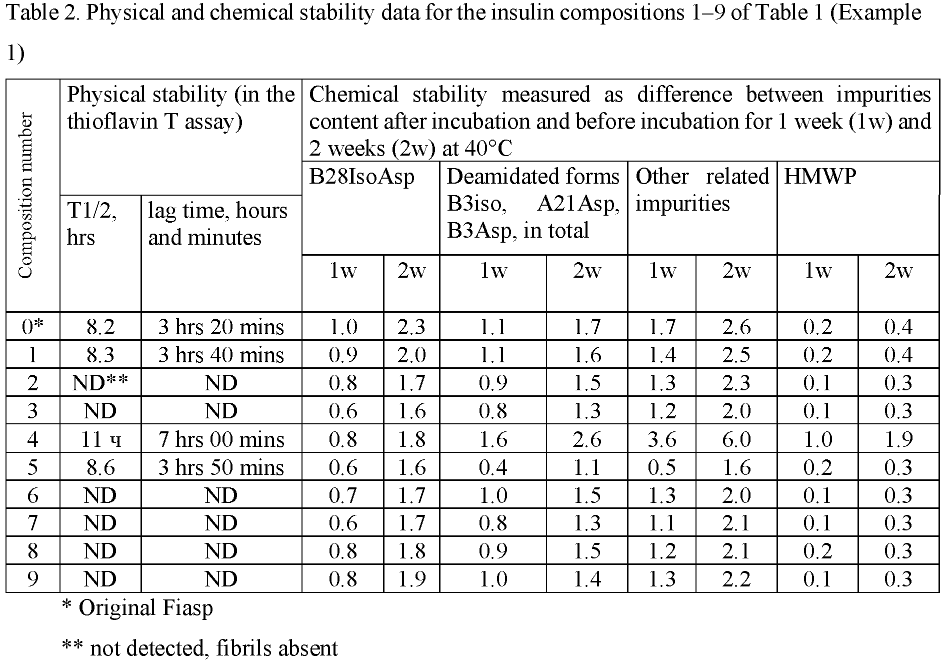

Based on the chemical and physical stability studies for the insulin aspart compositions prepared with either phosphate buffer or Tris-buffer, it was unexpectedly found that adding lysine or arginine, and/or salts thereof, and/or combination thereof combined with surfactants such as poloxamer 188, polysorbate 20, polysorbate 80, or combinations thereof, results in significant and reliable decrease in impurities comprised in the claimed compositions (compositions 2, 3, 6-9) after 2 weeks of storage at 40°C as compared to the prior art; another essential advantage of the claimed compositions is the enhanced physical stability, *i.e.,* the lack of tendency to fibrillation. The compositions of the invention showed no fibril formation (ND), however, if the amino acid concentration in the compositions exceeds 100 mM, their physical stability deteriorates, and the tendency to fibrillation comes up.

Compositions 1-9 produced by the method described in Example 1 were tested for physical stability in acceleration test. Three 3/16 inch (4.7625 mm) Teflon beads are placed into each of 2 mL glass HPLC vials. The vials are filled to capacity with samples of the tested compositions. The sealed vials are shaken continuously at 40 Hz (average linear acceleration 20 x g) with a 12 mm amplitude at 37°C, thereby subjecting the produced compositions to relatively high thermal and mechanical stress, *i.e.,* conditions favoring physical instability. The vials are placed onto vibrating mixer in such a way that in their long dimension (from top to bottom) they are parallel with the direction of linear acceleration, in other words, they are laid on their sides on the mixer surface. For other insulin compositions, increased stability under described acceleration conditions has been shown to correlate with significantly enhanced stability of the compositions during use. Turbidity (optical density at 450 nm) of the test samples and controls is regularly measured using a spectrophotometer. The control samples are produced by the same method as the test samples, but stored at 2-8°C without shaking. The resulting optical density value is calculated by subtracting the control sample optical density from the test sample optical density. Average values of the resulting optical densities and standard deviations for a number of samples are listed in Table 3.

**Table 3. Effects of the compositions' formulation and time of exposure to mechanical energy at 37° C on turbidity (optical density at 450 nm)**

| Composition number | Optical density at 450 nm | | | |
|---|---|---|---|---|
| | 24 h | 72 h | 120 h | 336 h |
| 1 | 0.85±0.73 (n=5) | Not determined | Not determined | Not determined |
| 2 | 0.02±0.01 (n=5) | 0.03±0.01 (n=5) | 0.02±0.01 (n=5) | 0.02±0.01 (n=5) |
| 3 | 0.02±0.01 (n=5) | 0.03±0.01 (n=5) | 0.02±0.01 (n=5) | 0.02±0.01 (n=5) |
| 4 | 0.02±0.01 (n=5) | 0.03±0.01 (n=5) | 0.03±0.01 (n=5) | 0.04±0.01 (n=5) |
| 5 | 0.02±0.01 (n=5) | 0.80±0.62 (n=5) | Not determined | Not determined |
| 6 | 0.03±0.01 (n=5) | 0.02±0.01 (n=5) | 0.03±0.01 (n=5) | 0.03±0.01 (n=5) |
| 7 | 0.02±0.01 (n=5) | 0.03±0.01 (n=5) | 0.02±0.01 (n=5) | 0.02±0.01 (n=5) |
| 8 | 0.03±0.01 (n=5) | 0.02±0.01 (n=5) | 0.04±0.01 (n=5) | 0.03±0.01 (n=5) |
| 9 | 0.02±0.01 (n=5) | 0.02±0.01 (n=5) | 0.02±0.01 (n=5) | 0.02±0.01 (n=5) |

In the above experimental conditions, turbidity of the resulting compositions, which lack combination of a surfactant and an amino acid (Composition 1), or lack a surfactant only (Composition 5), reaches high levels, becoming unacceptable by 24 hours for Composition 1, and by 72 hours for Composition 5, respectively. The optical density of all compositions comprising surfactants and amino acids stays almost the same as that of the controls during the 336 h experiment. The optical density of Composition 4 also stays almost the same as that of the controls, but the content of chemical degradation impurities in this composition is significantly higher compared to the samples comprising the combination of surfactants and amino acids.

Based on the observations made for the finished products of other insulins, one can assume that the unexpectedly and significantly increased stability of insulin compositions comprising combination of a surfactant and an amino acid, observed in acceleration test, will hold up in actual use for a period longer than 336 hours, since the compositions are exposed to a greater impact in the performed acceleration test compared to the use in injection pens and infusion systems.

### References:

1. Lougheed W. D., Woulfe-Flanagan H., Clement J. R., Albisser A. M. Insulin aggregation in artificial delivery systems. Diabetologia. 1980, 19(1), 1-9. doi:10.1007/bf00258302
2. Fisher H., Porter P. B. Pharmaceutical. Pharmacology. 1980, 33:203-206.
3. Irsigler K., Kritz H. Long-Term Continuous Intravenous Insulin Therapy with a Portable Insulin Dosage-regulating Apparatus. Diabetes. 1979, 28(3): 196-203. doi:10.2337/diab.28.3.196
4. Weisenfeld S., Podolsky S. Goldsmith, L. Ziff L. Adsorption of Insulin to Infusion Bottles and Tubing. Diabetes. 1968, 17(12): 766-771. doi:10.2337/diab.17.12.766
5. Browe et al. European Journal of Biochemistry. 1973, 33:233.
6. Selivanova O. M., Grishin S. Yu., Glyakina A. V. et al. Analiz analogov insulina i strategiya ikh dal'neishei razrabotki [Analysis of insulin analogues and strategy for their further development]. Uspekhi biologicheskoi khimii [Advances in biological chemistry]. 2018, 58: 313-346 (in Russian).
7. Brange J., Owens D. R., Kang S., Volund A. Monomeric Insulins and Their Experimental and Clinical Implications. Diabetes Care. 1990, 13(9), 923-954. doi:10.2337/diacare.13.9.923
8. Schlein M. Insulin Formulation Characterization - the Thioflavin T Assays. AAPS Journal. 2017, 19(2): 397 - 408.
9. Instruktsiya po primeneniyu lekarstvennogo preparata dlya meditsinskogo primeneniya «Fiasp» [Guideline for medical use of pharmaceutical preparation "Fiasp"]. URL: http://grls.rosminzdrav.ru (accessed on 30 Aug 2022) (in Russian).
10. Brange J. Stability of Insulin. Kluwer Academic Publisher. 1994, 18-23.
11. Paquot N., Scheen A. J. Faster aspart insulin (FIASP®). Revue Medicale de Liege. 2018; 73 (4): 211-215.
12. Muchmore D. B. Pump Users Clamor for Faster Insulin: Is Fast-Acting Insulin Aspart Ready for Them? Journal of Diabetes Science and Technology. 2018; 12(1): 152-154. doi:10.1177/1932296817750166
13. Ahem T. J., Manning M. C. Stability of Protein Pharmaceuticals. Plenum Press. New York. 1992.

## Claims

1. A composition for reducing blood glucose level in a subject, the composition comprising an insulin aspart (B28Asp), a nicotinic acid compound, one or more amino acids or a salt thereof selected from lysine, arginine or salts thereof; and a surfactant.

2. The composition of claim 1, wherein the composition comprises one or more surfactants selected from poloxamer 188, polysorbate 20 and polysorbate 80.

3. The composition of claim 1, wherein the insulin aspart (B28Asp) is present in an amount from about 0.2 mM (33 U/ml) to about 2 mM (333 U/ml).

4. The composition of claim 1, wherein the insulin aspart is present in an amount from about 0.3 mM (50 U/ml) to about 1.2 mM (200 U/ml).

5. The composition of claim 1, wherein the nicotinic acid compound is nicotinamide.

6. The composition of claim 1, wherein the composition comprises from about 1 mM to about 200 mM nicotinamide.

7. The composition of claim 1, wherein the composition comprises from about 1 mM to about 100 mM lysine or a salt thereof.

8. The composition of claim 1, wherein the composition comprises from about 1 mM to about 100 mM arginine or a salt thereof.

9. The composition of claim 1, wherein the composition comprises from about 1 mM to about 100 mM of a combination of arginine or a salt thereof with lysine or a salt thereof.

10. The composition of claim 1, wherein the composition comprises one or more surfactants selected from poloxamer 188, polysorbate 20 and polysorbate 80 in a concentration of about 0.001 to 0.05% w/v.

11. The composition of claim 1, wherein the composition further comprises a metal ion, preservative(s), isotonic agent(s) and stabilizer(s), buffer(s), and acidity regulators.

12. The composition of any of the claims 1-11 for use in the treatment or prevention of hyperglycemia, type 2 diabetes mellitus, impaired glucose tolerance, and type 1 diabetes mellitus.

13. An injectable insulin preparation for reducing blood glucose level in a subject, the preparation comprising the composition according to any of the claims 1-11.
